# EUROPÄISCHE PATENTSCHRIFT

(11) **EP 1 108 390 B1**
(45) Veröffentlichungstag und Bekanntmachung des Hinweises auf die Patenterteilung: **12.10.2005**
(21) Anmeldenummer: 00250429.8
(22) Anmeldetag: 12.12.2000
(51) Int. Cl.: A61B 5/0468, A61B 5/0452

(54) **Vorrichtung zur Erkennung der Kreislaufwirkungen von Extrasystolen**
Device for recognition of effect of extrasystols on the circulatory system
Dispositif de reconnaissance de l'effet des extrasystoles sur le système circulatoire

(30) Priorität: 17.12.1999 DE 19963246
(43) Veröffentlichungstag der Anmeldung: 20.06.2001
(73) Patentinhaber: BIOTRONIK Mess- und Therapiegeräte GmbH & Co Ingenieurbüro Berlin, 12359 Berlin (DE); Cortronik Mess- und Therapiegeräte GmbH, 8010 Graz (AT)
(72) Erfinder: Hutten, Helmut, Prof. Dr., 8010 Graz (AT)
(74) Vertreter: Eisenführ, Speiser & Partner

(56) Entgegenhaltungen:
- DE-A- 19 749 393
- US-A- 4 023 564
- HOIA P -W: "COMPUTER ARRHYTHMIA ANALYSIS IN AN EXERCISE SYSTEM" PROCEEDINGS OF THE ANNUAL CONFERENCE ON ENGINEERING IN MEDICINE AND BIOLOGY. NOT TO BE CONFUSED WITH PROCEEDINGS OF THE ANNUAL INTERNATIONAL CONFERENCE OF THE ENGINEERING IN MEDICINE AND BIOLOGY SOCIETY,US,NEW YORK, IEEE, Bd. CONF. 38, 30. September 1985 (1985-09-30), Seite 65 XP002038891

## Beschreibung

Die Erfindung betrifft eine kardiologische Vorrichtung mit einem Sensor, ausgebildet zur Aufnahme mindestens eines Herzsignals, sowie mit Signalverarbeitungsmitteln, die mit dem Sensor verbundene erste Erfassungsmittel umfassen, ausgebildet zum Erfassen eines Einzelsignals oder eines Merkmalparameters des Herzsignals:

Aus dem Stand der Technik sind Vorrichtungen bekannt, welche mindestens einen Sensor aufweisen, um aus dem damit erhaltenen elektrophysiologischen Meßsignal Rückschlüsse über die Tätigkeit des Herzens zu gewinnen.

Die bisherigen Vorrichtungen gehen davon aus, daß das Auftreten einer normalen Eigenerregung des Herzens als unmittelbarer und zeitbezogener Hinweis auf die Funktionstüchtigkeit des Herzens als mechanische Pumpe zur Aufrechterhaltung der Kreislaufstabilität angesehen werden kann. Extrasystolen sind Herzaktionen, die nicht im normalen Rhythmus der Herztätigkeit auftreten. Sie werden nach dem Ort der Erregungsbildung in supraventrikuläre bzw. atriale Extrasystolen und in ventrikuläre Extrasystolen unterteilt und unter dem Aspekt der Häufigkeit ihres Auftretens betrachtet. Ein gehäuftes Auftreten läßt diagnostische Rückschlüsse zu. In modernen elektrischen Herzschrittmachern sind Vorrichtungen zur Erkennung von Extrasystolen vorhanden, um sie bei der aufgabengerechten und bestimmungsgemäßen Ersatzfunktion der Herzstimulation zu berücksichtigen.

Eine weitergehende Untersuchung der Wirkung, die kardiologische Ereignisse wie Extrasystolen auf den Kreislauf und dessen Verhalten unmittelbar nach ihrem Auftreten haben, wird von den bekannten Vorrichtungen nicht vorgenommen.

Aus DE 197 49 393 ist eine Vorrichtung bekannt, die RR-Intervalle, die nach einer ventrikulären Extrasystole auftreten, mit einem Mittelwert der RR-Intervalle vor dem Auftreten der Extrasystole vergleicht.

Der Erfindung liegt die Aufgabe zugrunde, eine Vorrichtung anzugeben, die in der Lage ist, die Wirkung cardiophysiologischer Ereignisse wie Extrasystolen zu erfassen oder solche Ereignisse bereits vor Auftreten anzuzeigen. Insbesondere ist es die Aufgabe, eine Vorrichtung zu schaffen, die geeignet ist, die Auswirkung von Extrasystolen auf das Kreislaufsystem einschließlich des Herzens und auf die Tätigkeit der das Kreislaufsystem kontrollierenden physiologischen Systeme zu erfassen und weitergehende Informationen zu liefern, die zur Diagnose, zum Risikomonitoring, zur Unterstützung der Therapieführung und zur Verbesserung elektrischer Geräte, insbesondere elektrischer Herzschrittmacher verwendet werden können.

Diese Aufgabe wird erfindungsgemäß durch eine Vorrichtung der eingangs genannten Art gelöst, deren Signalverarbeitungsmittel zusätzlich umfassen:
- mit den ersten Erfassungsmitteln verbundene Mittelwertbildungsmittel, ausgebildet zum Bilden eines Mittelwertes über mehrere Werte des Merkmalparameters oder über mehrere Einzelsignale,
- mit dem Sensor verbundene zweite Erfassungsmittel, ausgebildet zum Erfassen von kardiologischen Ereignissen, insbesondere von Extrasystolen
- mit den zweiten Erfassungsmitteln, den Mittelwertbildungsmitteln und den ersten Erfassungsmitteln verbundene erste Vergleichsmittel, die zum Ermitteln einer Abweichung wenigstens eines im unmittelbaren zeitlichen Zusammenhang mit einem Ereignis wie einer Extrasystole ermittelten Einzelsignals oder Merkmalparameters von dem entsprechenden Mittelwert ausgebildet ist.

Weiterhin umfassen die Signalverarbeitungsmittel erfindungsgemäß zusätzlich zweite Vergleichsmittel, die mit den ersten Vergleichsmitteln verbunden und zum Vergleichen der Abweichung mit einem Grenzwert ausgebildet sind, sowie mit den zweiten Vergleichsmitteln verbundene Signalmittel, die zum Ausgeben eines Signals, falls die Differenz den Grenzwert überschreitet, ausgebildet sind.

Als das oder die in unmittelbarem zeitlichen Zusammenhang mit dem Ereignis stehende Einzelsignal oder -signale oder als der in unmittelbarem zeitlichen Zusammenhang mit dem Ereignis stehende Merkmalsparameter werden bevorzugt dasjenige Einzelsignal oder der- oder diejenigen Merkmalsparameter verwendet, der oder die unmittelbar auf das Ereignis folgen.

Unter "Herzsignal" oder "Einzelsignal" soll hier insbesondere ein Signalabschnitt beispielsweise eines EKG-Signals verstanden werden, wie er zwischen zwei periodisch wiederkehrenden Signalmerkmalen, z.B. einander entsprechenden Nulldurchgängen auftritt. Ein EKG-Signal besteht in diesem Sinne aus einer Kette von Einzelsignalen, die durch periodisch wiederkehrende Signalmerkmale voneinander abgegrenzt sind.

Das Erfassen und Speichern solcher Signalabschnitte bzw. Einzelsignale wie auch die Bildung von Mittelwerten über mehrere Signalabschnitte anhand vorgegebener zeitlicher Bewertungsfunktionen ist bereits in der nicht vorveröffentlichten DE 199 38 376 desselben Erfinders beschrieben. Die dort vorgesehenen Mittel und Methoden finden ihre Anwendung auch bei der hier beschriebenen Vorrichtung.

Die Erfindung schließt die Erkenntnis ein, daß bei der Erkennung der Wirkung von Extrasystolen auf den Kreislauf und sein Verhalten auch solche Zusammenhänge beachtet werden müssen, welche über eine unmittelbare Zuordnung zur mechanischen Leistung des Herzens hinausgehen. Insbesondere berücksichtigt die Erfindung die gesicherte Erkenntnis, daß sich individuelle Besonderheiten wie Form und Größe des Herzens, der Ort der Entstehung für die mit Extrasystolen verbundene elektrophysiologische Erregung des Herzens und pathophysiologische Veränderungen des Kreislaufsystems auf den Verlauf des Meßsignales auswirken können.

Ferner liegt der Erfindung die Erkenntnis zugrunde, daß in einem biologischen System unmittelbar nacheinander auftretende Signale niemals die genau gleiche Signalfeinstruktur aufweisen, sondern gewisse Abweichungen auftreten können, deren Ursache nicht ausschließlich mit dem Auftreten von Extrasystolen in Verbindung stehen müssen.

Extrasystolen sind Herzerregungen, die früher auftreten, als im normalen Herzrhythmus unter Berücksichtigung anderer Ursachen wie beispielsweise Atmung oder Schwankungen der Aktivität des autonomen Nervensystems zu erwarten ist. Die unmittelbare Wirkung von Extrasystolen kann vielfältig sein. Eine ventrikuläre Extrasystole kann so früh im Ablauf des normalen Herzzyklus auftreten, daß durch die zu diesem Zeitpunkt noch nicht abgeschlossene Kontraktion des Vorhofes der Ventrikel nur unvollständig gefüllt ist. Je nach dem Ort, an dem die ventrikuläre Extrasystole entsteht, kann auch der zeitlich koordinierte Ablauf der ventrikulären Kontraktion beeinflußt sein. In vielen Fällen kommt es zu einem mehr oder weniger verminderten Schlagvolumen und damit in weiterer Folge zu veränderten Zustandsgrößen im Herz-Kreislaufsystem, die durch vorhandene physiologische Sensoren, z.B. die Pressorezeptoren im Aortenbogen und im Carotissinus, festgestellt werden und zu Rückwirkungen auf das Verhalten des Herz-Kreislaufsystems führen. Auch atriale Extrasystolen können wegen ihres im Vergleich zum normalen Herzrhythmus verfrühten Auftretens und der dadurch nicht vollständigen Füllung des Ventrikels kreislaufwirksam sein. Im allgemeinen sind atriale Extrasystolen dadurch ausgezeichnet, daß die anschließende Pause bis zur nächsten normalen Systole (sog. postextrasystolische Pause) länger ist als die im normalen Herzrhythmus auftretende Pause. Als Folge der verlängerten Pause kann die Füllung des Ventrikels und damit das Schlagvolumen nach einer Extrasystole eher etwas größer werden, zugleich kann es jedoch wegen der längeren Pause zu einer stärkeren Entleerung der unmittelbar hinter dem Herzen liegenden Gefäßabschnitte mit Auswirkungen auf den dort herrschenden intravasalen Druck kommen. Ferner können als Folge kreislaufwirksamer Fluktuationen Auswirkungen auf unterschiedliche Hormonsysteme entstehen. Zusätzlich wirkt der Frank-Starling-Mechanismus, der einen in den Eigenschaften des Herzmuskels begründeten Zusammenhang zwischen der Füllung des Herzens und dem Schlagvolumen beschreibt. Die verschiedenen durch Extrasystolen hervorgerufenen Wirkungen zeichnen sich durch unterschiedliche Zeitkonstanten aus, die sich im zeitlichen Übergangsverhalten des Herz-Kreislaufsystems bis zum Erreichen des ursprünglichen Zustandes widerspiegeln.

In technischer Betrachtungsweise stellenkreislaufwirksame Extrasystolen kurzzeitige Störungen in einem System mit Mehrfachregelung dar. Die meisten der durch die Störung aktivierten Untersysteme wirken kompensatorisch im Sinne einer Wiederherstellung des Ausgangszustandes.

Bei den zur Erkennung der Kreislaufwirksamkeit von Extrasystolen herangezogenen Signalen handelt es sich um elektrische Signale, die in bekannter Weise mit einem Sensor gewonnen werden, welcher mit einer zur Vor- und Weiterverarbeitung dieser Signale geeigneten Vorrichtung in leitender Verbindung steht. Die Vorverarbeitung, z. B. die Verstärkung und frequenzbestimmende Filterung zur Befreiung von solchen Signalanteilen, die im Sinne der Erkennung der Kreislaufwirksamkeit nicht informationsrelevant sind, kann in einer dem Stand der Technik entsprechenden Weise erfolgen. Einige der dem Stand der Technik entsprechenden implantierbaren Herzschrittmacher mit Sensingkanal sind zu dieser Vorverarbeitung befähigt. Die Vorverarbeitung kann in analoger oder in digitaler Form ausgeführt werden.

Der Erfindung liegt eine Vorrichtung zugrunde, die es ermöglicht, aus einer Folge von elektrophysiologischen Signalen, die durch Herzerregungen unmittelbar nach einer oder mehrerer Extrasystolen hervorgerufen werden, nach geeigneter Vorverarbeitung solche Merkmalsparameter zu extrahieren, welche die Beurteilung der durch Extrasystolen bewirkten Beeinflussung des Kreislaufverhaltens gestatten. Die Merkmalsextraktion wird vorteilhafterweise in digitaler Verarbeitung durchgeführt, ist jedoch grundsätzlich auch in analoger oder gemischter analog-digitaler Verarbeitungsweise möglich.

Erfindungsgemäß wird die Aufgabe durch eine Vorrichtung verwirklicht, die mit der Fähigkeit zur Mittelwertbildung aus den in zeitlicher Folge nacheinander auftretenden Einzelsignalen ausgestattet ist. Die Mittelwertbildung kann für den gesamten Verlauf der Einzelsignale, soweit dieser für die Beurteilung des Kreislaufverhaltens relevant ist, oder für bestimmte Merkmale, die aus den Einzelsignalen gewonnen worden sind und für die Beurteilung des Kreislaufverhaltens relevant sind, durchgeführt werden. Bei den aus den Einzelsignalen gewonnenen Merkmalen kann es sich um ein Merkmal oder um mehrere Merkmale handeln.

Die Mittelwertbildung und die darauf beruhende Streuwertbildung erfordern für jedes Einzelsignal einen geeigneten, für den betrachteten Signalverlauf oder Merkmalsparameter charakteristischen Zeitbezug. Hierfür werden bevorzugt der erste im Zusammenhang mit der Ventrikeldepolarisation auftretende Signaldurchlauf durch die Linie des elektrischen Nullsignales oder das im Zusammenhang mit der Ventrikeldepolarisation auftretende Maximum verwendet. Vorrichtungen zur Bestimmung des charakteristischen Zeitbezugspunktes für jedes Einzelsignal an sich sind aus dem Stand der Technik bekannt.

Der Ermittlung des Mittelwertes kann eine zeitliche Bewertungsfunktion zugrunde gelegt werden. Durch die Mittelwertbildung wird eine ausreichende Berücksichtigung individueller Besonderheiten verwirklicht. Durch die zeitliche Bewertungsfunktion ermöglicht die Vorrichtung ferner eine fortlaufende Anpassung an Veränderungen des Signalverlaufes, die durch andere Einflüsse als Extrasystolen bewirkt werden.

Ferner besitzt die Vorrichtung die Fähigkeit, aus der Abweichung der Einzelsignale oder der daraus erhaltenen Merkmale vom Mittelwert einen Streuwert zu bilden. Dieser Streuwert ist ein statistisches Maß für die Abweichung der Einzelsignale oder der daraus erhaltenen Merkmale vom Mittelwert infolge der physiologischen Einflußfaktoren, zu denen die Extrasystolen nicht gehören.

Extrasystolen und eine nachfolgende Anzahl von Einzelsignalen, die im Erfindungsanspruch entsprechend dem Stand der Technik festgelegt wird, werden weder bei der Bildung des Mittelwertes noch bei der Berechnung des Streuwertes berücksichtigt. Da die Ermittlung des Mittelwertes und des Streuwertes mit Ausnahme der durch das Auftreten von Extrasystolen gekennzeichneten Intervalle fortlaufend vorgenommen wird, wird die im Sinne des Erfindungsanspruches wesentliche Anforderung der zeitlichen Adaptation an sich verändernde Verhältnisse, die Auswirkungen auf die Signalfeinstruktur haben, jedoch in keinem Zusammenhang mit den durch Extrasystolen ausgelösten Kreislaufwirkungen stehen, erfüllt.

Jedes in dem einer oder mehreren Extrasystolen nachfolgenden Intervall auftretende Einzelsignal wird mit dem bis zum Auftreten der Extrasystole gespeicherten Mittelwert verglichen. Dieser Vergleich kann bei gespeicherten Signalverläufen bevorzugt durch Verfahren wie Kreuzkorrelation, bei gespeicherten Merkmalsparametern durch Ermittlung der Differenz vorgenommen werden. Die hierfür einzusetzenden Verfahren und Vorrichtungen entsprechen dem Stand der Technik. Hierbei wird ein Wert ermittelt, der in einfacher quantitativer Weise die Abweichung des jeweiligen Einzelsignales oder des daraus extrahierten Merkmalsparameters vom gespeicherten Mittelwert beschreibt.

Das Ergebnis ist eine Folge positiver und negativer Zahlen, die die Abweichung der nach einer oder mehreren Extrasystolen auftretenden Einzelsignale oder der daraus extrahierten Merkmalsparameter von dem entsprechenden Mittelwert in ihrer zeitlichen Abfolge beschreibt. Damit werden die durch die oder mehreren Extrasystolen hervorgerufenen Wirkungen auf das Herz-Kreislaufsystem in ihrem zeitlichen Verlauf bis zum Wiedererreichen des Ausgangszustandes dargestellt.

Die weitergehende Bewertung der so ermittelten Zahlenfolge kann in unterschiedlicher Weise erfolgen, z.B. durch einen bewertenden Vergleich mit einer in einer Look-Up-Tabelle gespeicherten Zahlenfolge oder durch Approximation der Zahlenfolge mit Funktionen höherer Ordnung.

Die weitergehende Bewertung und die Umsetzung des dadurch erhaltenen Ergebnisses sind insofern nicht Gegenstand des Erfindung, wie die daraus zu ziehenden diagnostischen Erkenntnisse oder therapeutischen Schlußfolgerungen die Anwendung der erfindungsgemäßen Vorrichtung bei einer größeren Zahl von Patienten voraussetzen.

Die Erfindung besteht somit insbesondere in einer Vorrichtung zur Erkennung der Kreislaufwirkung, die durch Extrasystolen hervorgerufen wird, wobei die Vorrichtung Merkmalsparameter, die für bestimmte Kreislauf zustände kennzeichnend sind, auf der Basis einer fortlaufenden Bildung von Mittelwerten mit beliebig einstellbarer zeitlicher Bewertungsfunktion ermittelt, mit den nach einer oder mehrerer Extrasystolen auftretenden Einzelsignalen vergleicht und die Abweichung von einem Grenzwert auf der Basis des Streuwertes, der ebenfalls von der erfindungsgemäßen Vorrichtung fortlaufend ermitteltwird, als Entscheidungskriterium für eine Kreislaufwirkung verwendet.

Vorteilhafte Ausgestaltungen dieser Vorrichtung bestehen darin:
- daß als Merkmalsparameter zur Erkennung der Kreislaufwirkung als Folge einer oder mehrerer Extrasystolen das Zeitintervall zwischen zwei unmittelbar aufeinanderfolgenden Herzerregungen, ermittelt aus den Zeitpunkten des gleichartigen Nulldurchganges des mit dem Sensor (1) festgestellten elektrophysiologischen Signales verwendet wird.
- daß als Merkmalsparameter zur Erkennung der Kreislaufwirkung als Folge einer oder mehrerer Extrasystolen das Zeitintervall zwischen zwei unmittelbar aufeinanderfolgenden Herzerregungen, ermittelt aus den Zeitpunkten des Auftretens des Signalmaximums des mit dem Sensor (1) festgestellten elektrophysiologischen Signales verwendet wird.
- daß als Merkmalsparameter zur Erkennung der Kreislaufwirkung als Folge einer oder mehrerer Extrasystolen das Maximum des mit dem Sensor (1) festgestellten elektrophysiologischen Signales verwendet wird.
- daß als Merkmalsparameter zur Erkennung der Kreislaufwirkung als Folge einer oder mehrerer Extrasystolen der Kreuzkorrelationskoeffizient zwischen dem Mittelwertsignal und dem aktuell aufgetretenen Einzelsignal verwendet wird.
- daß mehrere Merkmalsparameter ermittelt und in zusammenfassender Bewertung als Kriterium zur Erkennung der Kreislaufwirkung als Folge einer oder mehrerer Extrasystolen verwendet werden.
- daß die Merkmalsparameter zur Erkennung der Kreislaufwirkung als Folge einer oder mehrerer Extrasystolen während eines Intervalles bestimmt werden, das durch eine Zeitvorgabe festgelegt wird.
- daß die Merkmalsparameter zur Erkennung der Kreislaufwirkung als Folge einer oder mehrerer Extrasystolen während eines Intervalles bestimmt werden, das durch eine Anzahl von normalen Herzerregungen nach der auslösenden Extrasystole festgelegt wird.
- daß die Merkmalsparameter zur Erkennung der Kreislaufwirkung als Folge einer oder mehrerer Extrasystolen während eines Intervalles bestimmt werden, das durch einen aus dem Streuwert abgeleiteten Schwellwert bestimmt wird.
- daß der Sensor (1) zur Erfassung des elektrophysiologischen Signales der Herzerregung noch andere Funktionen wie die Stimulation des Herzens wahrnehmen kann.
- daß der Sensor (1) zur Erfassung des elektrophysiologischen Signales der Herzerregung aus mehreren Komponenten besteht.
- daß die Vorrichtung in einem dafür geeigneten Gehäuse vollständig im Körper eines Menschen implantiert wird.
- daß die Vorrichtung gemeinsam mit einem elektrischen Herzschrittmacher in einem Gehäuse untergebracht und vollständig im Körper eines Menschen implantiert wird.
- daß die Vorrichtung dazu verwendet wird, die Stimulationsfrequenz eines elektrischen Herzschrittmachers in geeigneter Weise an die Kreislauferfordernisse des Patienten anzupassen.

Da in der erfindungsgemäßen Vorrichtung bei Ermitteln einer oder mehrerer Extrasystolen auch der Signalverlauf vor einer oder mehrerer Extrasystolen gespeichert ist, kann die Vorrichtung auch geringfügig dahingehend abgewandelt werden, daß unmittelbar vor einer oder mehrerer Extrasystolen aufgenommene Einzelsignale oder Merkmalsparameter mit den entsprechenden Mittelwerten aus zuvor aufgenommen regulären Signalverläufen in der hier oder in der DE 199 38 376 beschriebenen Art verglichen und analysiert werden, um vor Auftreten einer (weiteren) Extrasystole ein entsprechendes Signal ausgeben zu können.

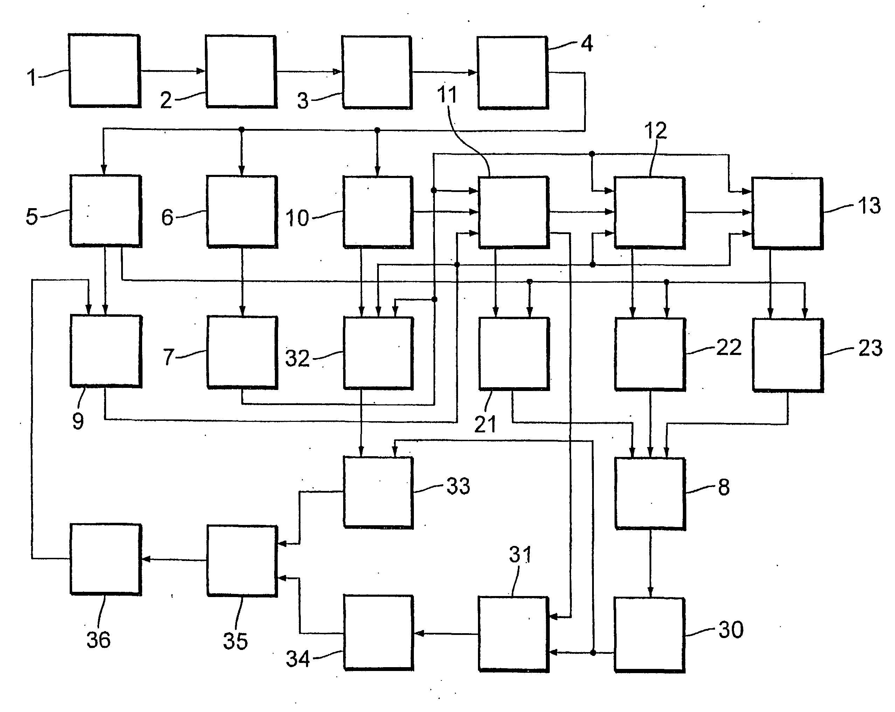

Figur 1 zeigt die wesentlichen Funktionseinheiten und ihre funktionsgerechte Anordnung, mit der der Oberbegriff des Anspruches verwirklicht werden kann. Die im Text in Klammern zu den Funktionseinheiten aufgeführten Ziffern entsprechen den in Figur 1 zur Bezeichnung der Funktionseinheiten verwendeten Ziffern.

Das mit dem Sensor (1) aufgenommene Signal als Ausdruck der Herzerregung wird nach geeigneter Vorverarbeitung, die im vorliegenden Beispiel in analoger Form mit einem Eingangsverstärker (2) und einem Filter (3) erfolgt, einem Analog-Digital-Wandler (4) zugeführt. Die Analog-Digital-Wandlung muß mit einer ausreichend hohen Abtastrate und mit ausreichender Amplitudendiskretisierung vorgenommen werden, so daß dadurch keine Verfälschung bzw. Elimination jener Signalanteile bewirkt wird, die für die Erkennung der von einer oder mehrerer Extrasystolen hervorgerufenen Wirkungen auf das Herz-Kreislaufsystem wesentlich sind.

Das auf diese Weise erhaltene digitalisierte Signal wird mehreren Funktionseinheiten zugeführt:
1. einer Vorrichtung zur Erkennung, daß eine oder mehrere Extrasystolen vorliegt und welcher Klasse sie zuzuordnen ist (5);
2. einer Vorrichtung zur Erkennung des charakteristischen Zeitbezugspunktes in jedem Einzelsignal für die Mittelwert- und Streuwertbildung (6);
3. einem ersten Speicher (10), dessen Kapazität ausreichend sein muß, um jedes Einzelsignal mit der für die erfindungsgemäße Aufgabenstellung erforderlichen Zeit- und Amplitudendiskretisierung aufzunehmen.

Hinter dem Speicher (10) sind N weitere und gleichartig aufgebaute Speicher (11, 12... 1N) angeordnet, deren Speicherkapazität jener des ersten Speichers (10) entsprechen muß. Die Zahl N der in dieser Weise angordneten gleichartig aufgebauten Speicher kann beliebig groß Bein, muß jedoch mindestens gleich 2 sein.

Mit dem Auftreten eines Einzelsignales wird der Inhalt des ersten Speichers (10) in den unmittelbar nachfolgenden Speicher (11), dessen Inhalt in den unmittelbar nachfolgenden Speicher (12) usw. übernommen. Lediglich der Inhalt des letzten Speichers (1 N) wird nicht übernommen, sondern geht verloren. Die Übernahme des Inhaltes jedes Speichers in den nachfolgenden Speicher erfolgt geordnet und koordiniert durch einen Taktgenerator (7) in der Weise, daß in entsprechenden Speicherelementen jedes Speichers die in Bezug auf den charakteristischen Zeitbezugspunkt entsprechenden Elemente der verschiedenen Einzelsignale stehen.

Der Taktgenerator wird dazu durch ein Steuersignal der Vorrichtung zur Erkennung des charakteristischen Zeitbezugspunktes (6) aktiviert.

Die in den hintereinander angeordneten Speichern (11, 12... 1N) enthaltenen und einander entsprechenden Signalanteile werden dann, wenn das in den Speicher (10) übernommene nächste Einzelsignal keine Extrasystole ist, was durch die Vorrichtung zur Erkennung von Extrasystolen (5) festgestellt wird oder in das der Extrasystole folgende und festgelegte Intervall fällt, über Bewertungsvorrichtungen (21, 22... 2N) einer Addiervorrichtung (8) zugeführt. Dabei ist jede der Bewertungsvorrichtungen in gleicher Weise mit einem der Speicher verbunden, z.B. der zweite Speicher (11) mit der Bewertungsvorrichtung (21), der dritte Speicher (12) mit der Bewertungsvorrichtung (22) usw. Insgesamt sind also ebenso viele Bewertungsvorrichtungen wie Speicher mit Ausnahme des ersten Speichers (10) vorhanden. Allerdings kann jede Bewertungsvorrichtung mit einem eigenen Bewertungsfaktor zwischen 1 und 0 mit geeigneter Unterteilung ausgestattet werden. Damit kann der Inhalt jedes Speichers (11, 12... 1 N) mit einem einstellbaren Bewertungsfaktor multiplikativ gewichtet werden, bevor er der Addiervorrichtung zugeführt wird (8). Der Addiervorrichtung nachgeschaltet ist ein weiterer Speicher (30) der wie die Speicher (11, 12... 1N) aufgebaut ist. Der Inhalt dieses Speichers (30) entspricht also nach durchgeführter Addition einem Signal, das den mit dem Auftreten jedes Einzelsignales neu gebildeten und auf der Basis einer beliebig einstellbaren Gewichtungsfunktion sowie des charakteristischen Zeitbezugswertes ermittelten Mittelwert der in den Speichern (11, 12... 1N) enthaltenen Signale darstellt.

Wenn die Vorrichtung zur Erkennung von Extrasystolen (5) das Auftreten einer oder mehrerer Extrasystole feststellt, wird über die Intervall-Vorgabevorrichtung (9) die Signalübernahme in der Speicheranordnung (10, 11, 12.. 1N) in den jeweils folgenden Speicher inhibiert. Die Inhibierung bleibt für die gesamte Dauer jenes der oder mehrerer Extrasystolen folgenden Intervalles wirksam, während dessen die von der ode mehrerer Extrasystolen hervorgerufenen Kreislaufwirkungen andauern. Damit befinden sich während dieses Intervalles in den Speichern (11, 12,...1N) in geordneter Reihenfolge die N der oder mehrerer Extrasystolen unmittelbar vorausgehenden normalen Systolen. Der gewichtete Mittelwert der N der oder mehrerer Extrasystolen unmittelbar vorausgehenden normalen systolen im Mittelwertpeicher (30) bleibt somit während dieses Intervalles unverändert.

Da die in einem biologischen System nacheinander auftretenden Einzelsignale niemals identisch sind, wird zusätzlich aus den in den Speichern (11, 12... 1N) gespeicherten Signalen in der Streuwert-Ermittlungsvorrichtung (31) durch Vergleich des im Speicher (11) enthaltenen Einzelsignales mit dem im Mittelwertspeicher (30) enthaltenen Mittelwertsignal ein Streuwert gebildet. Dieser Streuwert stellt ein Maß für die normale bzw. physiologische Streubreite der Einzelsignale, die normalen Erregungsvorgängen entsprechen, um jenen Mittelwert dar, der fortlaufend und gewichtet ermittelt wird. Vorrichtungen zur Ermittlung des Streuwertes sind bekannt und entsprechen dem Stand der Technik. Bevorzugt werden hierfür Mikroprozessoren verwendet.

Während der durch die Vorrichtung (9) festgelegten Dauer des Intervalles, in der die Inhalte der Speicher (11, 12... 1N) unverändert bleiben, wird in derselben Weise, in der außerhalb dieses Intervalles die Übernahme aus dem ersten Speicher (10) in den nachfolgenden Speicher (11) erfolgt, der Inhalt des ersten Speichers (10) in den Speicher (32) übernommen. Der bis zu diesem Zeitpunkt im Speicher (32) abgelegte Inhalt geht dabei verloren. Im Speicher (32) steht somit jeweils das zuletzt aufgetretene, welches in das durch die Extrasystole ausgelöste Intervall fällt.

In der Vorrichtung (33) wird die Abweichung des Inhaltes des Speichers (32) vom Inhalt des Mittelwertspeichers (30) quantitativ ermittelt. Der Vergleich der beiden Signale kann sich sowohl auf den gesamten Signalverlauf als auch auf jedes Merkmal beziehen, das für den Zustand bestimmter Kreislaufparameter kennzeichnend ist. Bei diesen Merkmale kann es sich um zeitliche Merkmale, z. B. das Auftreten charakteristischer Punkte im Signalverlauf wie der erste Durchgang durch die elektrische Nulllinie mit beginnender Ventrikeldepolarisation oder das während der Depolarisation festzustellende Maximum, um Zeitdifferenzen, z.B. um die Dauer des positiven Signalverlaufes bei Depolarisation, oder um Amplitudenwerte, z.B. die Größe des während der Depolarisation festzustellenden Maximums, handeln.

Die in der Vorrichtung (33) zur quantitativen Ermittlung der Abweichung zwischen dem Inhalt des Mittelwertspeichers (30) und dem Inhalt des Speichers (32) festgestellte Abweichung wird in Beziehung zu dem bei normaler Herztätigkeit auftretenden Streuwert gesetzt. Dazu wird in der Vergleichsschaltung (35) der in der Vorrichtung (33) ermittelte Wert der Abweichung mit einem Wert verglichen, der in der Vorrichtung (34) aus dem durch die Streuwert-Ermittlungsvorrichtung (31) ermittelten Streuwert gebildet worden ist. Kreislaufwirkung als Folge der oder mehrerer aufgetretener Extrasystolen liegt dann vor, wenn die Vergleichsschaltung (35) einen Unterschied zwischen diesen beiden Wert feststellt. Die von der Vorrichtung (35) erzeugte Zahlenfolge steht für die weitergehende Auswertung für diagnostische Bewertung, Erkennung von Risikoentwicklung, Therapieunterstützung oder Steuerung medizintechnischer Geräte zur Verfügung.

Da das Fehlen eines auf Kreislaufwirkung hinweisenden Unterschiedes zufällig bedingt sein kann, z.B. bei einer oszillierenden Fluktuation bei Nulldurchgang, wird durch Vorrichtung (36) überprüft, ob die Nullbedingung bei k aufeinanderfolgenden Herzerregungen erfüllt ist, wobei k beliebig groß sein kann und mindestens gleich 2 sein muß.

## Patentansprüche

1. Kardiologische Vorrichtung mit
einem Sensor (1), ausgebildet zur Aufnahme mindestens eines Herzsignals sowie mit Signalverarbeitungsmitteln (2, 3, 4), die mit dem Sensor (1) verbundene erste Erfassungsmittel (10) umfassen, ausgebildet zum Erfassen eines Einzelsignals oder eines Merkmalparameters des Herzsignals,
wobei
die Signalverarbeitungsmittel weiterhin umfassen:
- mit den ersten Erfassungsmitteln verbundene Mittelwertbildungsmittel (11, 12, 13, 21, 22, 23, 8, 30), ausgebildet zum Bilden eines Mittelwertes über mehrere Werte des Merkmalparameters oder über mehrere Einzelsignale,
- mit dem Sensor verbundene zweite Erfassungsmittel (5), ausgebildet zum Erfassen von kardiologischen Ereignissen, insbesondere von Extrasystolen,
- mit den zweiten Erfassungsmitteln (5), den Mittelwertbildungsmitteln (11, 12, 13, 21, 22, 23, 8, 30) sowie den ersten Erfassungsmitteln (10) verbundene erste Vergleichsmittel (32, 33), die zum Ermitteln einer Abweichung eines im unmittelbaren zeitlichen Zusammenhang mit mindestens einem Ereignis wie einer Extrasystole oder einer Folge von Extrasystolen ermittelten Einzelsignals oder Merkmalparameters von dem entsprechenden Mittelwert ausgebildet sind,
**gekennzeichnet durch**
- mit den ersten Vergleichsmitteln (32, 33) verbundene zweite Vergleichsmittel (35), ausgebildet zum Vergleichen der Abweichung mit einem Grenzwert
- mit den zweiten Vergleichsmitteln verbundene Signalmittel (35), ausgebildet zum Ausgeben eines Signals, wenn die Abweichung den Grenzwert überschreitet.

2. Vorrichtung nach Anspruch 1, **gekennzeichnet durch** Streuwertermittlungsmittel (31), die mit den ersten Erfassungsmitteln (10) verbunden und derart ausgebildet sind, daß sie einen Streuwert für den oder die Merkmalsparameter bilden.

3. Vorrichtung nach Anspruch 1 und 2, **gekennzeichnet durch** Grenzwertbildungsmittel (34), die mit den Streuwertermittlungsmitteln (31) verbunden und so ausgebildet sind, daß Grenzwert aus der Basis des Streuwertes gebildet wird.

4. Vorrichtung nach einem der Ansprüche 1 bis 3, **gekennzeichnet durch** Inhibitormittel, die mit den zweiten Erfassungsmitteln (5) und mit den Mittelwertbildungsmitteln (11, 12, 13, 21, 22, 23, 8, 30) verbunden und so ausgebildet sind, daß wenigstens ein im unmittelbaren zeitlichen Zusammenhang mit einem Ereignis wie einer Extrasystole auftretendes Einzelsignal oder ein entsprechender Merkmalsparameter nicht in die Mittelwertbildung eingeht.

5. Vorrichtung nach einem der Ansprüche 1 bis 4, **dadurch gekennzeichnet, daß** die ersten Vergleichsmittel (32, 33) so ausgebildet sind, daß sie unmittelbar nach einem Ereignis wie einer Extrasystole ermittelte Einzelsignale oder Merkmalsparameter mit dem entsprechenden Mittelwert vergleichen.

6. Vorrichtung nach einem der Ansprüche 1 bis 5, **dadurch gekennzeichnet, daß** die Mittelwertbildungsmittel (11, 12, 13, 21, 22, 23, 8, 30) so ausgebildet sind, daß die Mittelwertbildung fortlaufend anhand einer beliebig einstellbaren zeitlichen Bewertungsfunktion erfolgt.

7. Vorrichtung nach einem der Ansprüche 1 bis 6, **dadurch gekennzeichnet, daß** die ersten Erfassungsmittel (9, 10, 11) derart ausgebildet sind, daß die ersten Erfassungsmittel (09, 10, 11) den oder die Merkmalparameter jeweils innerhalb eines vorgebbaren Intervalls bestimmen.

8. Vorrichtung nach Anspruch 7, **dadurch gekennzeichnet, daß** die ersten Erfassungsmittel (10) mit den Streuwertermittlungsmitteln (31) verbunden und derart ausgebildet sind, daß sie das Intervall durch einem aus dem Streuwert abgeleiteten Schwellwert bestimmen.

9. Vorrichtung nach einem der Ansprüche 1 bis 8, **dadurch gekennzeichnet, daß** der von den ersten Erfassungsmitteln (10) erfaßte Merkmalsparameter oder die erfaßten Merkmalsparameter sind: die Herzrate, gemessen von einem Signalmaximum zum nächsten oder vom einem Nullgang zum nächsten Nulldurchgang gleicher Art, das Signalmaximum oder der Kreuzkorrelationskoeffizient aufeinanderfolgender Einzelsignale oder eine Kombination dieser Merkmalsparameter.

## Claims

1. A cardiological device comprising
a sensor (1) adapted to pick up at least one cardiac signal and signal processing means (2, 3, 4) which include first detection means (10) connected to the sensor (1), adapted to detect an individual signal or a feature parameter of the cardiac signal,
wherein
the signal processing means further include:
- averaging means (11, 12, 13, 21, 22, 23, 8, 30) connected to the first detection means, adapted to form an average over a plurality of values of the feature parameter or over a plurality of individual signals,
- second detection means (5) connected to the sensor, adapted to detect cardiological events, in particular extrasystoles,
- first comparison means (32, 33) which are connected to the second detection means (5), the averaging means (11, 12, 13, 21, 22, 23, 8, 30) and the first detection means (10) and which are adapted to ascertain a deviation of a feature parameter or individual signal ascertained in direct time relationship with at least one event such as an extrasystole or a sequence of extrasystoles, from the corresponding average,
**characterised by**
- second comparison means (35) connected to the first comparison means (32, 33), adapted to compare the deviation to a limit value, and
- signal means (35) connected to the second comparison means, adapted to output a signal when the deviation exceeds the limit value.

2. A device according to claim 1 **characterised by** dispersion value ascertaining means (31) which are connected to the first detection means (10) and which are so adapted that they form a dispersion value for the feature parameter or parameters.

3. A device according to claim 1 or claim 2 **characterised by** limit value forming means (34) which are connected to the dispersion value ascertaining means (31) and which are so adapted that the limit value is formed on the basis of the dispersion value.

4. A device according to one of claims 1 to 3 **characterised by** inhibitor means which are connected to the second detection means (5) and to the averaging means (11, 12, 13, 21, 22, 23, 8, 30) and which are so adapted that at least one individual signal occurring in direct time relationship with an event such as an extrasystole or a corresponding feature parameter is not involved in the averaging operation.

5. A device according to one of claims 1 to 4 **characterised in that** the first comparison means (32, 33) are so adapted that they compare feature parameters or individual signals ascertained immediately after an event such as an extrasystole to the corresponding average.

6. A device according to one of claims 1 to 5 **characterised in that** the averaging means (11, 12, 13, 21, 22, 23, 8, 30) are so adapted that the averaging operation is effected continuously on the basis of any adjustable time assessment function.

7. A device according to one of claims 1 to 6 **characterised in that** the first detection means (9, 10, 11) are so adapted that the first detection means (9, 10, 11) determine the feature parameter or parameters within a respective predeterminable interval.

8. A device according to claim 7 **characterised in that** the first detection means (10) are connected to the dispersion value ascertaining means (31) and are so adapted that they determine the interval by a threshold value derived from the dispersion value.

9. A device according to one of claims 1 to 8 **characterised in that** the feature parameter or feature parameters detected by the first detection means (10) are: the heart rate measured from one signal maximum to the next or from one zero-crossing to the next zero-crossing of the same kind, the signal maximum or the cross-correlation coefficient of successive individual signals or a combination of those feature parameters.

## Revendications

1. Dispositif de cardiologie comportant
un capteur (1) agencé de manière à enregistrer au moins un signal cardiaque ainsi que des moyens (2,3,4) de traitement de signaux, qui comportent des premiers moyens de détection (10) reliés au capteur (1) et agencés pour détecter un signal individuel et un paramètre caractéristique du signal cardiaque,
dans lequel
les moyens de traitement de signaux comprennent en outre :
- des moyens (11,12,13,21,22,23,8,30) de formation de valeur moyenne reliés aux premiers moyens de détection et agencés pour former une valeur moyenne sur plusieurs valeurs du paramètre caractéristique ou sur plusieurs signaux individuels,
- des seconds moyens de détection (5) reliés au capteur et agencés pour détecter des événements cardiologiques, notamment des extrasystoles,
- des premiers moyens comparateurs (32,33), qui sont reliés aux seconds moyens de détection (5), aux moyens (11,12,13,21,22,23,8,30) de formation de la valeur moyenne, ainsi qu'aux premiers moyens de détection (10) et qui sont agencés pour déterminer un écart d'un signal individuel ou d'un paramètre caractéristique, déterminé dans une relation temporelle directe avec au moins un événement tel qu'une extrasystole ou une suite d'extrasystoles, par rapport à la valeur moyenne correspondante,
**caractérisé par**
- des seconds moyens comparateurs (35), qui sont reliés aux premiers moyens comparateurs (32,33) et servent à comparer l'écart par rapport à une valeur limite,
- des moyens (35) de délivrance de signaux, qui sont reliés aux seconds moyens comparateurs et sont agencés pour délivrer un signal lorsque l'écart dépasse la valeur limite.

2. Dispositif selon la revendication 1, **caractérisé par** des moyens (31) de détermination de valeurs aberrantes, qui sont reliés aux premiers moyens de détection (10) et sont agencés de telle sorte qu'ils forment une valeur aberrante pour le ou les paramètres caractéristiques.

3. Dispositif selon les revendications 1 et 2, **caractérisé par** des moyens (34) de formation de valeur limite, qui sont reliés aux moyens (31) de détermination de valeurs aberrantes et sont agencés de telle sorte que la valeur limite est formée sur la base de la valeur aberrante.

4. Dispositif selon l'une des revendications 1 à 3, **caractérisé par** des moyens d'inhibition, qui sont reliés aux seconds moyens de détection (5) et aux moyens (11,12, 13,21,22,23,8,30) de formation de la valeur moyenne et sont agencés de telle sorte qu'au moins un signal individuel, qui apparaît selon une liaison temporelle directe avec l'événement comme une extrasystole, ou un paramètre caractéristique correspondant n'intervient pas dans la formation de la valeur moyenne.

5. Dispositif selon l'une des revendications 1 à 4, **caractérisé en ce que** les premiers moyens comparateurs (32, 33) sont agencés de telle sorte qu'ils comparent directement des signaux individuels ou des paramètres caractéristiques, déterminés directement après un événement tel qu'une extrasystole, à la valeur moyenne correspondante.

6. Dispositif selon l'une des revendications 1 à 5, **caractérisé en ce que** les moyens (11,12,13,21,22,23,8,30) de formation de la valeur moyenne sont agencés de telle sorte que la formation de la valeur moyenne s'effectue de façon continue en référence à une fonction de pondération temporelle réglable à volonté.

7. Dispositif selon l'une des revendications 1 à 6, **caractérisé en ce que** les premiers moyens de détection (9, 10,11) sont agencés de telle sorte que les premiers moyens de détection (9,10,11) déterminent le ou les paramètres caractéristiques respectivement à l'intérieur d'un intervalle pouvant être prédéterminé.

8. Dispositif selon la revendication 7, **caractérisé en ce que** les premiers moyens de détection (10) sont reliés aux moyens (31) de détermination de valeurs aberrantes et sont agencés de telle sorte qu'ils déterminent l'intervalle au moyen d'une valeur de seuil dérivée de la valeur aberrante.

9. Dispositif selon l'une des revendications 1 à 8, **caractérisé en ce que** le paramètre caractéristique détecté par les premiers moyens de détection (10) ou les paramètres caractéristiques détectés sont : le rythme cardiaque mesuré depuis un maximum du signal jusqu'au maximum suivant ou depuis un passage par zéro jusqu'au passage par zéro suivant de même type, le maximum du signal ou le coefficient de corrélation croisée de signaux individuels successifs ou une combinaison de ces paramètres caractéristiques.
